# EUROPÄISCHE PATENTSCHRIFT

(11) **EP 0 923 562 B1**
(45) Veröffentlichungstag und Bekanntmachung des Hinweises auf die Patenterteilung: **09.10.2002**
(21) Anmeldenummer: 97934551.9
(22) Anmeldetag: 28.07.1997
(51) Int. Cl.: C07D 239/54, C07D 409/12, C07C 271/28, A01N 43/54

(54) **SUBSTITUIERTE P-TRIFLUORMETHYLPHENYLURACILE**
SUBSTITUTED P-TRIFLUOROMETHYLPHENYLURACILS
P-TRIFLUORMETHYLPHENYLURACILES SUBSTITUES

(30) Priorität: 08.08.1996 DE 19632005
(43) Veröffentlichungstag der Anmeldung: 23.06.1999
(73) Patentinhaber: BAYER AG, 51368 Leverkusen (DE)
(72) Erfinder: DREWES, Mark-Wilhelm, D-40764 Langenfeld (DE); ANDREE, Roland, D-40764 Langenfeld (DE); DOLLINGER, Markus, D-51381 Leverkusen (DE)
(86) Internationale Anmeldenummer: EP9704082
(87) Internationale Veröffentlichungsnummer: WO98006706

(56) Entgegenhaltungen:
- EP-A- 0 438 209
- DE-A- 19 524 617
- CHEMICAL ABSTRACTS, vol. 119, no. 11, 1993 Columbus, Ohio, US; abstract no. 117269m, Seite 936; Spalte 2; XP002047013 in der Anmeldung erwähnt & JP 00 525 142 A (NISSAN) 2.Februar 1993 in der Anmeldung erwähnt

## Beschreibung

Die Erfindung betrifft neue substituierte p-Trifluormethylphenyluracile, Verfahren zu ihrer Herstellung und ihre Verwendung als Herbizide.

Es ist bekannt, daß bestimmte substituierte p-Trifluormethylphenyluracile, wie z.B. die Verbindung 3-(3-Chlor-4-trifluormethyl-phenyl)-6-(2,6-difluor-phenyl)-[1H,3H]-pyrimidin-2,4-dion pestizide Eigenschaften aufweisen, d.h. gegen bestimmte tierische Schädlinge, insbesondere gegen Insekten und Milben wirksam sind (vgl. JP 05025142 - zitiert in Chem. Abstracts 119:117269, EP-A-0 438 209, US 3,580,913, US 3,869,457). Über eine herbizide Wirksamkeit dieser Verbindungen ist jedoch nichts bekannt geworden.

Es wurden nun die neuen substituierten p-Trifluormethylphenyluracile der allgemeinen Formel (I) 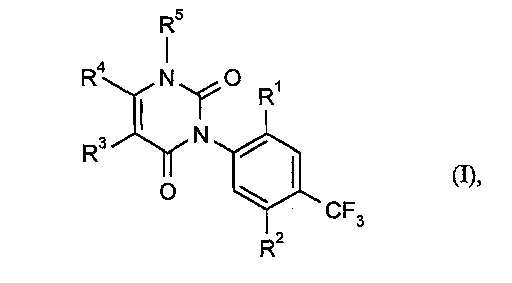 in welcher
- R¹: für Wasserstoff, Cyano, Fluor, Chlor oder Brom steht,
- R²: für die Gruppierung A¹-A²-A³ steht, worin
A¹ für die Gruppierung -N-A⁴- steht, worin A⁴ für Wasserstoff, Hydroxy, C₁-C₄-Alkyl, C₁-C₄-Alkoxy, Phenyl, C₁-C₄-Alkylsulfonyl oder Phenyl-sulfonyl steht,
A² für eine Einfachbindung, für O, S, -SO-, -SO₂-, -CO- oder die Gruppierung -N-A⁴- steht, worin A⁴ für Wasserstoff, Hydroxy, C₁-C₄-Alkyl, C₁-C₄-Alkoxy, Phenyl, C₁-C₄-Alkylsulfonyl oder Phenylsulfonyl steht,
A² weiterhin für jeweils gegebenenfalls durch Fluor oder Chlor substituiertes C₁-C₆-Alkandiyl, C₂-C₆-Alkendiyl, C₂-C₆₋Alkindiyl, C₃-C₆-Cycloalkandiyl, C₃-C₆-Cycloalkendiyl oder Phenylen steht,
A³ für Wasserstoff, Hydroxy, Mercapto, Amino, Cyano, Isocyanato, Thiocyanato, Nitro, Carboxy, Carbamoyl, Thiocarbamoyl, Sulfo, Chlorsulfonyl, Halogen, für jeweils gegebenenfalls durch Fluor, Chlor, Brom oder C₁-C₄-Alkoxy substituiertes Alkyl, Alkoxy, Alkylthio, Alkylsulfinyl, Alkylsulfonyl, Alkylamino, Dialkylamino, Alkoxycarbonyl oder Dialkoxy(thio)phosphoryl mit jeweils 1 bis 6 Kohlenstoffatomen in den Alkylgruppen, für jeweils gegebenenfalls durch Fluor, Chlor oder Brom substituiertes Alkenyl, Alkenyloxy, Alkenylamino, Alkylidenamino, Alkenyloxycarbonyl, Alkinyl, Alkinyloxy, Alkinylamino oder Alkinyloxycarbonyl mit jeweils 2 bis 6 Kohlenstoffatomen in den Alkenyl-, Alkyliden- oder Alkinylgruppen, für jeweils gegebenenfalls durch Fluor, Chlor, Brom, Cyano, Carboxy, C₁-C₄-Alkyl und/oder C₁-C₄-Alkoxy-carbonyl substituiertes Cycloalkyl, Cycloalkyloxy, Cycloalkylalkyl, Cycloalkylalkoxy, Cycloalkylidenamino, Cycloalkyloxycarbonyl oder Cycloalkylalkoxycarbonyl mit jeweils 3 bis 6 Kohlenstoffatomen in den Cycloalkylgruppen und gegebenenfalls 1 bis 4 Kohlenstoffatomen in den Alkylgruppen, oder für jeweils gegebenenfalls durch Nitro, Cyano, Carboxy, Fluor, Chlor, Brom, C₁-C₄-Alkyl, C₁-C₄-Halogenalkyl, C₁-C₄-Alkyloxy, C₁-C₄-Halogenalkyloxy und/oder C₁-C₄-Alkoxy-carbonyl substituiertes Phenyl, Phenyloxy, Phenyl-C₁-C₄-alkyl, Phenyl-C₁-C₄-alkoxy, Phenyloxycarbonyl oder Phenyl-C₁-C₄-alkoxycarbonyl, (jeweils gegebenenfalls ganz oder teilweise hydriertes) Pyrrolyl, Pyrazolyl, Imidazolyl, Triazolyl, Furyl, Thienyl, Oxazolyl, Isoxazolyl, Thiazolyl, Isothiazolyl, Oxadiazolyl, Thiadiazolyl, Pyridinyl, Pyrimidinyl, Triazinyl, Pyrazolyl-C₁-C₄-alkyl, Furyl-C₁-C₄-alkyl, Thienyl-C₁-C₄-alkyl, Oxazolyl-C₁-C₄-alkyl, Isoxazol-C₁-C₄-alkyl, Thiazol-C₁-C₄-alkyl, Pyridinyl-C₁-C₄-alkyl, Pyrimidinyl-C₁-C₄-alkyl, Pyrazolylmethoxy, Furylmethoxy, für Perhydropyranylmethoxy oder Pyridylmethoxy steht,
- R³: für Wasserstoff, Fluor, Chlor, Brom oder gegebenenfalls durch Fluor und/oder Chlor substituiertes Alkyl mit 1 bis 4 Kohlenstoffatomen steht,
- R⁴: für gegebenenfalls durch Fluor und/oder Chlor substituiertes Alkyl mit 1 bis 4 Kohlenstoffatomen steht, und
- R⁵: für Wasserstoff, Amino oder gegebenenfalls durch Hydroxy, Cyano, Fluor, Chlor oder C₁-C₄-Alkoxy substituiertes Alkyl mit 1 bis 4 Kohlenstoffatomen steht,
gefunden.

Man erhält die neuen substituierten p-Trifluormethylphenyluracile der allgemeinen Formel (I), wenn man

Aminoalkensäureester der allgemeinen Formel (II) 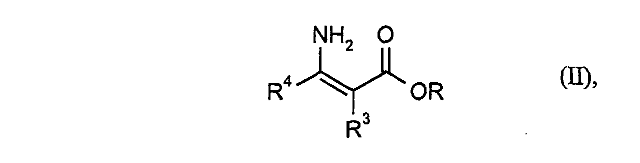 in welcher
- R³ und R⁴: die oben angegebenen Bedeutungen haben und
- R: für Alkyl, Aryl oder Arylalkyl steht,
mit p-Trifluormethylphenylisocyanaten der allgemeinen Formel (III) 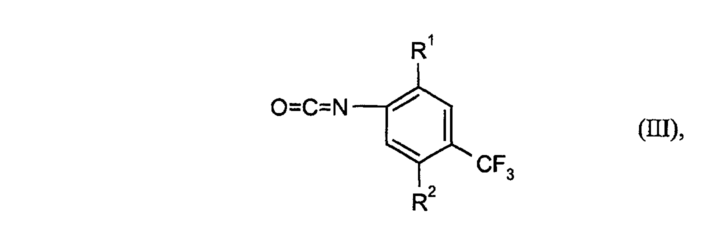 in welcher
- R¹ und R²: die oben angegebenen Bedeutungen haben,
oder mit p-Trifluormethylphenylurethanen (p-Trifluormethylphenylcarbamaten) der allgemeinen Formel (IV) 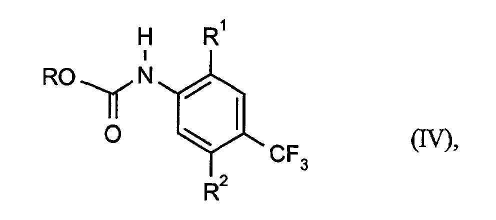 in welcher
- R¹ und R²: die oben angegebenen Bedeutungen haben und
- R: für Alkyl, Aryl oder Arylalkyl steht,
gegebenenfalls in Gegenwart eines Reaktionshilfsmittels und gegebenenfalls in Gegenwart eines Verdünnungsmittels umsetzt
und gegebenenfalls an den so erhaltenen Verbindungen der Formel (I) im Rahmen der obigen Substituentendefinitionen weitere Umwandlungen nach üblichen Methoden durchführt.

Die Verbindungen der allgemeinen Formel (I) können nach üblichen Methoden in andere Verbindungen der allgemeinen Formel (I) gemäß obiger Substituentendefinitionen umgewandelt werden, beispielsweise durch übliche elektrophile oder nucleophile Substitutionsreaktionen (z.B. R⁵: H → NH₂, CH₃).

Die neuen substituierten p-Trifluormethylphenyluracile der allgemeinen Formel (I) zeichnen sich durch starke herbizide Wirksamkeit aus. Sie zeigen darüber hinaus gute Verträglichkeit gegenüber wichtigen Kulturpflanzen, wie z.B. Weizen, Gerste und Mais.

In den Definitionen sind die gesättigten oder ungesättigten Kohlenwasserstoffketten, wie Alkyl, Alkenyl oder Alkinyl - auch in Verbindung mit Heteroatomen, wie in Alkoxy oder Alkylthio - jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Gegenstand der Erfindung sind vorzugsweise Verbindungen der Formel (I), in welcher
- R¹: für Wasserstoff, Fluor oder Chlor steht,
- R²: für die Gruppierung A¹-A²-A³ steht, worin
A¹ für die Gruppierung -N-A⁴- steht, worin A⁴ für Wasserstoff, Hydroxy, Methyl, Ethyl, n- oder i-Propyl, Methoxy, Ethoxy, n- oder i-Propoxy, Methylsulfonyl oder Ethylsulfonyl steht,
A² für eine Einfachbindung, für O, S, -SO-, -SO₂-, -CO- oder die Gruppierung -N-A⁴- steht, worin A⁴ für Wasserstoff, Hydroxy, Methyl, Ethyl, n- oder i-Propyl, Methoxy, Ethoxy, n- oder i-Propoxy, Methylsulfonyl, Ethylsulfonyl, n- oder i-Propylsulfonyl oder Phenylsulfonyl steht,
A² weiterhin für Methylen, Ethan-1,1-diyl, Ethan-1,2-diyl, Propan-1,1-diyl, Propan-1,2-diyl, Propan-1,3-diyl, Ethen-1,2-diyl, Propen-1,2-diyl, Propen-1,3-diyl, Ethin-1,2-diyl oder Propin-1,3-diyl steht,
A³ für Wasserstoff, Hydroxy, Amino, Cyano, Nitro, Carboxy, Carbamoyl, Sulfo, Fluor, Chlor, Brom, für jeweils gegebenenfalls durch Fluor, Chlor, Methoxy oder Ethoxy substituiertes Methyl, Ethyl, n- oder i- Propyl, n-, i-, s- oder t-Butyl, n-, i-, s- oder t-Pentyl, Methoxy, Ethoxy, n- oder i-Propoxy, n-, i-, s- oder t-Butoxy, n-, i-, s- oder t-Pentyloxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Dimethylamino, Diethylamino, Methoxycarbonyl, Ethoxycarbonyl, n- oder i-Propoxycarbonyl, Dimethoxyphosphoryl, Diethoxyphosphoryl, Dipropoxyphosphoryl oder Diisopropoxyphosphoryl, für jeweils gegebenenfalls durch Fluor oder Chlor substituiertes Propenyl, Butenyl, Propenyloxy, Butenyloxy, Propenylamino, Butenylamino, Propylidenamino, Butylidenamino, Propenyloxycarbonyl, Butenyloxycarbonyl, Propinyl, Butinyl, Propinyloxy, Butinyloxy, Propinylamino, Butinylamino, Propinyloxycarbonyl oder Butinyloxycarbonyl, für jeweils gegebenenfalls durch Fluor, Chlor, Cyano, Carboxy, Methyl, Ethyl, n- oder i-Propyl, Methoxycarbonyl oder Ethoxycarbonyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylmethyl, Cyclobutylmethyl, Cyclopentylmethyl, Cyclohexylmethyl, Cyclo-propylmethoxy, Cyclobutylmethoxy, Cyclopentylmethoxy, Cyclohexylmethoxy, Cyclopentylidenamino, Cyclohexylidenamino, Cyclopentyloxycarbonyl, Cyclohexyloxycarbonyl, Cyclopentylmethoxycarbonyl oder Cyclohexylmethoxycarbonyl, oder für jeweils gegebenenfalls durch Nitro, Cyano, Carboxy, Fluor, Chlor, Brom, Methyl, Ethyl, n- oder i- Propyl, Trifluormethyl, Methoxy, Ethoxy, n- oder i-Propoxy, Difluormethoxy, Trifluormethoxy, Methoxycarbonyl und/oder Ethoxycarbonyl substituiertes Phenyl, Phenyloxy, Benzyl, Phenylethyl, Benzyloxy, Phenyloxycarbonyl, Benzyloxycarbonyl, (jeweils gegebenenfalls ganz oder teilweise hydriertes) Pyrrolyl, Pyrazolyl, Imidazolyl, Triazolyl, Furyl, Thienyl, Oxazolyl, Isoxazolyl, Thiazolyl, Isothiazolyl, Oxadiazolyl, Thiadiazolyl, Pyridinyl, Pyrimidinyl, Triazinyl, Pyrazolylmethyl, Furylmethyl, Thienylmethyl, Oxazolylmethyl, Isoxazolmethyl, Thiazolmethyl, Pyridinylmethyl, Pyrimidinylmethyl, Pyrazolylmethoxy, Furylmethoxy oder Pyridylmethoxy steht,
- R³: für Wasserstoff, Fluor, Chlor, Brom oder jeweils gegebenenfalls durch Fluor und/oder Chlor substituiertes Methyl oder Ethyl steht,
- R⁴: für jeweils durch Fluor und/oder Chlor substituiertes Methyl oder Ethyl steht, und
- R⁵: für Wasserstoff, Amino oder jeweils gegebenenfalls durch Hydroxy, Cyano, Fluor, Chlor, Methoxy oder Ethoxy substituiertes Methyl oder Ethyl steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Beispiele für die erfindungsgemäßen Verbindungen der Formel (I) sind in den nachstehenden Gruppen aufgeführt.

### Gruppe 1

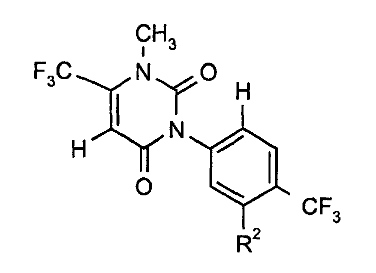

R² hat hierbei beispielhaft die in der nachstehenden Aufzählung angegebenen Bedeutungen:
Methylamino, Ethylamino, n- oder i-Propylamino, n-, i-, s- oder t-Butylamino, n-, i-, s-oder t-Pentylamino, Cyanomethylamino, Cyanoethylamino, Cyanopropylamino, Cyanobutylamino, Carboxymethylamino, Carboxyethylamino, Carboxypropylamino, Carboxybutylamino, Methoxymethylamino, Ethoxymethylamino, Propoxymethylamino, Methoxyethylamino, Ethoxyethylamino, Propoxyethylamino, Methoxypropylamino, Ethoxypropylamino, Propoxypropylamino, Methoxycarbonylmethylamino, Ethoxycarbonylmethylamino, Propoxycarbonylmethylamino, Methoxycarbonylethylamino, Ethoxycarbonylethylamino, Propoxycarbonylethylamino, Methoxycarbonylpropylamino, Ethoxycarbonylpropylamino, Propoxycarbonylpropylamino, 1-Propen-3-yl-amino (Allylamino), 3-Methyl-1-propen-3-ylamino, 2-Buten-4-yl-amino (Crotonylamino), 1-Propin-3-yl-amino (Propargylamino), 3-Methyl-1-propin-3-yl-amino, 2-Butin-4-yl-amino, Cyclopropylamino, Cyanocyclopropylamino, Carboxycyclopropylamino, Difluorcyclopropylamino, Dichlorcyclopropylamino, Methylcyclopropylamino, Methoxycarbonylcyclopropylamino, Ethoxycarbonylcyclopropylamino, Cyclobutylamino,
Cyanocyclobutylamino, Carboxycyclobutylamino, Difluorcyclobutylamino, Trifluorcyclobutylamino, Tetrafluorcyclobutylamino, Chlortrifluorcyclobutylamino, Methylcyclobutylamino, Cyclopentylamino, Cyanocyclopentylamino, Carboxycyclopentylamino, Fluorcyclopentylamino, Chlorcyclopentylamino, Difluorcyclopentylamino, Dichlorcyclopentylamino, Methylcyclopentylamino, Methoxycarbonylcyclopentylamino, Ethoxycarbonylcyclopentylamino, Cyclohexylamino, Cyanocyclohexylamino, Carboxycyclohexylamino, Fluorcyclohexylamino, Chlorcyclohexylamino, Difluorcyclohexylamino, Dichlorcyclohexylamino, Methylcyclohexylamino, Trifluormethylcyclohexylamino, Methoxycarbonylcyclohexylamino, Ethoxycarbonylcyclohexylamino, Cyclopropylmethylamino, Difluorcyclopropylmethylamino, Dichlorcyclopropylmethylamino, Cyclobutylmethylamino, Cyclopentylmethylamino, Cyclohexylmethylamino, Cyanocyclohexylmethylamino, Carboxycyclohexylmethylamino, Fluorcyclohexylmethylamino, Chlorcyclo-hexylmethylamino, Methylcyclohexylmethylamino, Trifluormethylcyclohexylmethylamino,
Phenylamino, Cyanophenylamino, Carboxyphenylamino, Nitrophenylamino, Fluorphenylamino, Chlorphenylamino, Bromphenylamino, Methylphenylamino, Trifluormethylphenylamino, Methoxyphenylamino, Difluormethoxyphenylamino, Trifluormethoxyphenylamino, Methoxycarbonylphenylamino,amino,amino, Cyanobenzylamino, Carboxybenzylamino, Fluorbenzylamino, Chlorbenzylamino, Methylbenzylamino, Trifluormethylbenzylamino, Methoxybenzylamino, Difluormethoxybenzylamino, Trifluormethoxybenzylamino, Methoxycarbonylbenzylamino, Ethoxycarbonylbenzylamino, Phenylethylamino. Methylsulfonylamino, Ethylsulfonylamino, n- oder i-Propylsulfonylamino, n-, i-, s- oder t-Butylsulfonylamino, N,N-(Bis-methylsulfonyl)-amino, N,N-(Bis-ethylsulfonyl)-amino, N-Methylsulfonyl-N-ethylsulfonyl-amino, N-Methylsulfonyl-N-propylsulfonyl-amino, N-Acetyl-N-methylsulfonylamino, N-Acetyl-N-ethylsulfonylamino, N-Propionyl-N-methylsulfonyl-amino, N-Propionyl-N-ethylsulfonyl-amino, N-n-Butyroyl-N-methylsulfonyl-amino, N-n-Butyroyl-N-ethylsulfonyl-amino, N-i-Butyroyl-N-methylsulfonyl-amino, N-i-Butyroyl-N-ethylsulfonylamino, N-n-Valeroyl-N-methylsulfonyl-amino, N-n-Valeroyl-N-ethylsulfonyl-amino, N-i-Valeroyl-N-methylsulfonyl-amino, N-i-Valeroyl-N-ethylsulfonyl-amino, N-s-Valeroyl-N-methylsulfonyl-amino, N-s-Valeroyl-N-ethylsulfonyl-amino, N-t-Butyl-carbonyl-N-methylsulfonyl-amino, N-t-Butyl-carbonyl-N-ethylsulfonyl-amino, N-Benzoyl-N-methylsulfonyl-amino, N-Benzoyl-N-ethylsulfonyl-amino.

### Gruppe 2

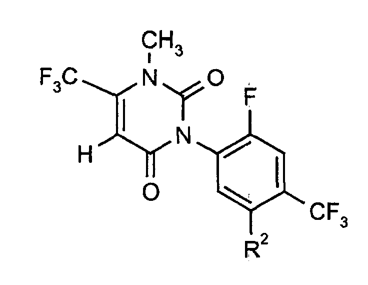

R² hat hierbei beispielhaft die oben in Gruppe 1 aufgeführten Bedeutungen.

### Gruppe 3

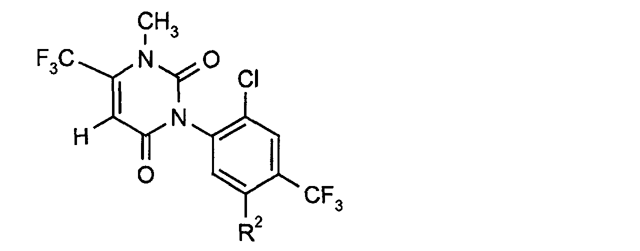

R² hat hierbei beispielhaft die oben in Gruppe 1 aufgeführten Bedeutungen.

### Gruppe 4

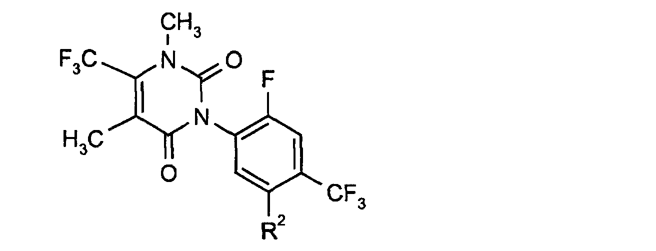

R² hat hierbei beispielhaft die oben in Gruppe 1 aufgeführten Bedeutungen.

### Gruppe 5

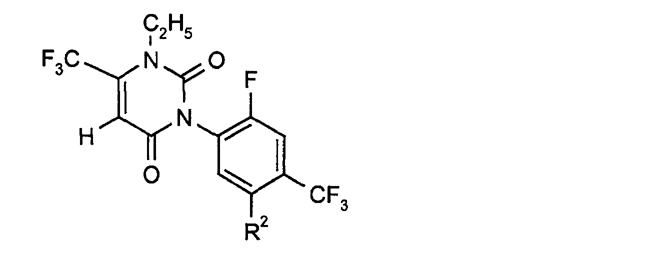

R² hat hierbei beispielhaft die oben in Gruppe 1 aufgeführten Bedeutungen.

### Gruppe 6

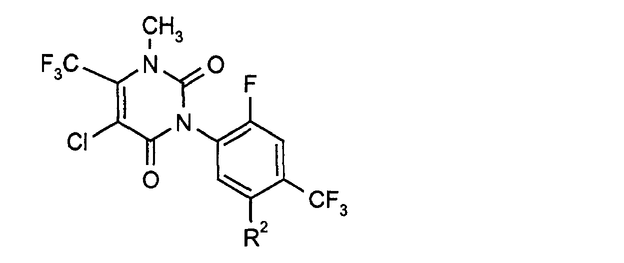

R² hat hierbei beispielhaft die oben in Gruppe 1 aufgeführten Bedeutungen.

### Gruppe 7

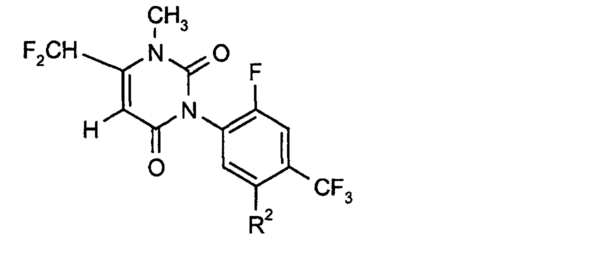

R² hat hierbei beispielhaft die oben in Gruppe 1 aufgeführten Bedeutungen.

### Gruppe 8

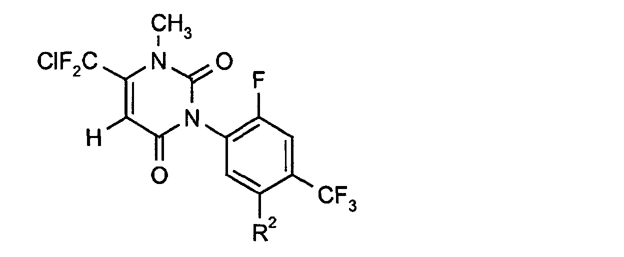

R² hat hierbei beispielhaft die oben in Gruppe 1 aufgeführten Bedeutungen.

### Gruppe 9

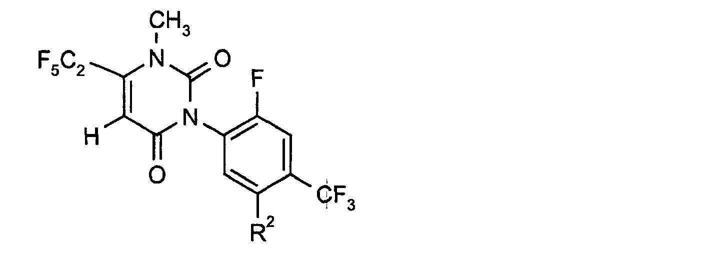

R² hat hierbei beispielhaft die oben in Gruppe 1 aufgeführten Bedeutungen.

### Gruppe 10

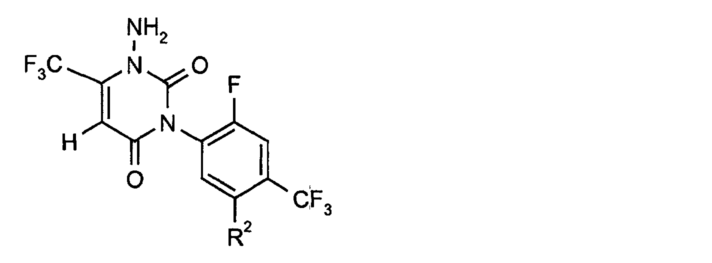

R² hat hierbei beispielhaft die oben in Gruppe 1 aufgeführten Bedeutungen.

Die beim erfindungsgemäßen Verfahren zur Herstellung von Verbindungen der Formel (I) als Ausgangsstoffe zu verwendenden Aminoalkensäureester sind durch die Formel (II) allgemein definiert. In der Formel (II) haben R³ und R⁴ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R³ und R⁴ angegeben wurden.

Die Ausgangsstoffe der Formel (II) sind bekannt und/oder können nach bekannten Verfahren hergestellt werden (vgl. J. Heterocycl. Chem. 9 (1972), 513-522).

Die beim erfindungsgemäßen Verfahren zur Herstellung von Verbindungen der Formel (I) weiter als Ausgangsstoffe zu verwendenden p-Trifluormethylphenylisocyanate sind durch die Formel (III) allgemein definiert. In der Formel (III) haben R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und R² angegeben wurden.

Die Ausgangsstoffe der Formel (III) sind bekannt und/oder können nach bekannten Verfahren hergestellt werden (vgl. EP 246061)

Man erhält die p-Trifluormethylphenylisocyanate der allgemeinen Formel (III), wenn man p-Trifluormethylaniline der allgemeinen Formel (V) 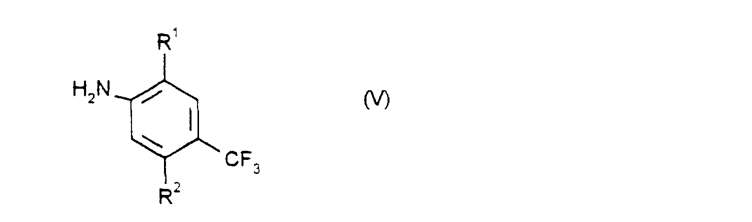 in welcher
R¹ und R² die oben angegebenen Bedeutungen haben,
mit Phosgen in Gegenwart eines Verdünnungsmittels, wie z.B. Chlorbenzol, bei Temperaturen zwischen -20°C und +150°C umsetzt (vgl. z.B. auch EP 648749).

Die beim erfindungsgemäßen Verfahren gegebenenfalls als Ausgangsstoffe zu verwendenden p-Trifluormethylphenylurethane sind durch die Formel (IV) allgemein definiert. In der Formel (IV) haben R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und R² angegeben wurden; R steht vorzugsweise fur C₁-C₄-Alkyl, Phenyl oder Benzyl, insbesondere für Methyl oder Phenyl.

Die Ausgangsstoffe der Formel (IV) sind noch nicht aus der Literatur bekannt; sie sind als neue Stoffe auch Gegenstand der vorliegenden Anmeldung.

Man erhält die neuen p-Trifluormethylphenylurethane der allgemeinen Formel (IV), wenn man p-Trifluormethylaniline der allgemeinen Formel (V) 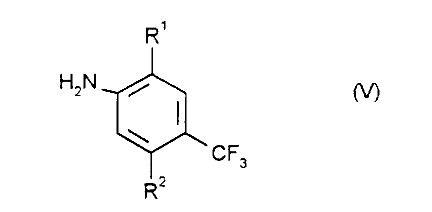 n welcher
R¹ und R² die oben angegebenen Bedeutungen haben,
mit Chlorcarbonylverbindungen der allgemeinen Formel (VI)

RO-CO-Cl (VI)

in welcher
R die oben angegebene Bedeutung hat,
gegebenenfalls in Gegenwart eines Säureakzeptors, wie z.B. Pyridin, und gegebenenfalls in Gegenwart eines Verdünnungsmittels, wie z.B. Methylenchlorid, bei Temperaturen zwischen -20°C und +100°C umsetzt (vgl. die Herstellungsbeispiele).

Als Reaktionshilfsmittel für das erfindungsgemäße Verfahren zur Herstellung von Verbindungen der Formel (I) kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -s- oder -t-butanolat, weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Als Verdünnungsmittel zur Durchführung des erfindungsgemäßen Verfahrens kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff, Ether, wie Diethylether, Diisopropylether, Dioxan, Tetrahydrofuran oder Ethylenglykoldimethyl- oder -diethylether, Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsauretriamid; Ester wie Essigsauremethylester oder Essigsaureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Die Reaktionstemperaturen können bei der Durchführung des erfindungsgemäßen Verfahrens in einem größeren Bereich variiert werden. Im allgemeinen arbeitet man bei Temperaturen zwischen 0°C und 200°C, vorzugsweise zwischen 10°C und 150°C.

Das erfindungsgemäße Verfahren wird im allgemeinen unter Normaldruck durchgeführt. Es ist jedoch auch möglich, das erfindungsgemaße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfruchtund Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im NachauflaufVerfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgierund/oder schaumerzeugende Mittel kommen in Frage: z.B. nichtionogene und anionische Emulgatoren, wie Polyoxyethylen-Fettsaure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Acetochlor, Acifluorfen(-sodium), Aclonifen, Alachlor, Alloxydim(-sodium), Ametryne, Amidochlor, Amidosulfuron, Asulam, Atrazine, Azimsulfuron, Benazolin, Benfuresate, Bensulfuron(-methyl), Bentazon, Benzofenap, Benzoylprop(ethyl), Bialaphos, Bifenox, Bromobutide, Bromofenoxim, Bromoxynil, Butachlor, Butylate, Cafenstrole, Carbetamide, Chlomethoxyfen, Chloramben, Chloridazon, Chlorimuron(-ethyl), Chlornitrofen, Chlorsulfuron, Chlortoluron, Cinmethylin, Cinosulfuron, Clethodim, Clodinafop(-propargyl), Clomazone, Clopyralid, Clopyrasulfuron, Cloransulam(-methyl), Cumyluron, Cyanazine, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Difenzoquat, Diflufenican, Dimefuron, Dimepiperate, Dimethachlor, Dimethametryn, Dimethenamid, Dinitramine, Diphenamid, Diquat, Dithiopyr, Diuron, Dymron, EPTC, Esprocarb, Ethalfluralin, Ethametsulfuron(-methyl), Ethofumesate, Ethoxyfen, Etobenzanid, Fenoxaprop-ethyl, Flamprop(-isopropyl), Flamprop(-isopropyl-L), Flamprop(methyl), Flazasulfuron, Fluazifop(-butyl), Flumetsulam, Flumiclorac(-pentyl), Flumioxazin, Flumipropyn, Fluometuron, Fluorochloridone, Fluoroglycofen(-ethyl), Flupoxam, Flupropacil, Flurenol, Fluridone, Fluroxypyr, Flurprimidol, Flurtamone, Fomesafen, Glufosinate(-ammonium), Glyphosate(-isopropylammonium), Halosafen, Haloxyfop(-ethoxyethyl), Hexazinone, Imazamethabenz(-methyl), Imazamethapyr, Imazamox, Imazapyr, Imazaquin, Imazethapyr, Imazosulfuron, Ioxynil, Isopropalin, Isoproturon, Isoxaben, Isoxaflutole, Isoxapyrifop, Lactofen, Lenacil, Linuron, MCPA, MCPP, Mefenacet, Metamitron, Metazachlor, Methabenzthiazuron, Metobenzuron, Metobromuron, Metolachlor, Metosulam, Metoxuron, Metsulfuron(-methyl), Metribuzin, Molinate, Monolinuron, Naproanilide, Napropamide, Neburon, Nicosulfuron, Norflurazon Orbencarb, Oryzalin, Oxadiazon, Oxyfluorfen, Paraquat, Pendimethalin, Phenmedipham, Piperophos, Pretilachlor, Primisulfuron(-methyl), Prometryn, Propachlor, Propanil, Propaquizafop, Propyzamide, Prosulfocarb, Prosulfuron, Pyrazolate, Pyrazosulfuron(-ethyl), Pyrazoxyfen, Pyributicarb, Pyridate, Pyrithiobac(-sodium), Quinchlorac, Quinmerac, Quizalofop(-ethyl), Quizalofop(-p-tefuryl), Rimsulfuron, Sethoxydim, Simazine, Simetryn, Sulcotrione, Sulfentrazone, Sulfometuron(-methyl), Sulfosate, Tebutam, Tebuthiuron, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele

### Beispiel 1

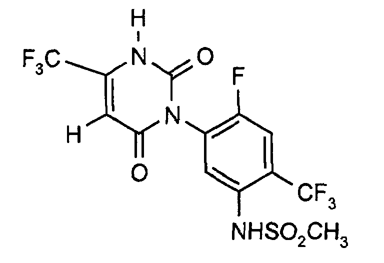

Eine Mischung aus 10 g (27,8 mMol) 1-(2,5-Difluor-4-trifluormethyl-phenyl)-3,6-dihydro-2,6-dioxo-4-trifluormethyl-1(2H)-pyrimidin, 5,8 g (27,8 mMol) Methansulfonsäureamid, 12 g Kaliumcarbonat und 40 ml N-Methyl-pyrrolidon wird ca. 35 Stunden bei 170°C gerührt. Nach Abkühlen auf Raumtemperatur wird die Mischung auf Eiswasser gegossen, mit 2N-Salzsäure angesäuert und mit Essigsäureethylester extrahiert. Die organische Phase wird mit Wasser gewaschen, mit Natriumsulfat getrocknet und filtriert. Das Filtrat wird im Wasserstrahlvakuum eingeengt, der Rückstand mit 0,5 ml Essigsäureethylester / 3 ml Diethylether digeriert und das kristalline Produkt durch Absaugen isoliert.

Man erhält 3,6 g (30% der Theorie) (27,8 mMol) 1-(2-Fluor-5-methylsulfonyl-4-trifluormethyl-phenyl)-3,6-dihydro-2,6-dioxo-4-trifluormethyl-1(2H)-pyrimidin vom Schmelzpunkt 227°C.

### Beispiel 2

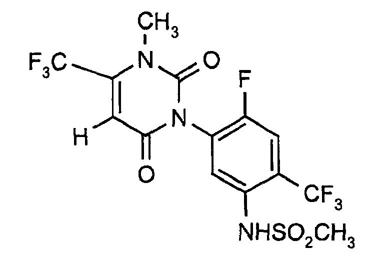

Eine Mischung aus 1,0 g (2,3 mMol) 1-(2-Fluor-5-methylsulfonyl-4-trifluormethylphenyl)-3,6-dihydro-2,6-dioxo-4-trifluormethyl-1(2H)-pyrimidin, 0,31 g (2,3 mMol) Dimethylsulfat, 0,21 g Natriumhydrogencarbonat und 20 ml Aceton wird 7 Stunden unter Rückfluß erhitzt und anschließend im Wasserstrahlvakuum eingeengt. Der Rückstand wird mit Diethylether und 2N-Salzsäure geschüttelt, die organische Phase abgetrennt, mit Wasser gewaschen, mit Natriumsulfat getrocknet und filtriert. Das Filtrat wird im Wasserstrahlvakuum eingeengt, der Rückstand mit Diethylether digeriert und das kristalline Produkt durch Absaugen isoliert.

Man erhält 0,62 g (60% der Theorie) 1-(2-Fluor-5-methylsulfonyl-4-trifluormethylphenyl)-3,6-dihydro-2,6-dioxo-3-methyl-4-trifluormethyl-1(2H)-pyrimidin vom Schmelzpunkt 163°C.

Analog zu den Herstellungsbeispielen 1 und 2 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

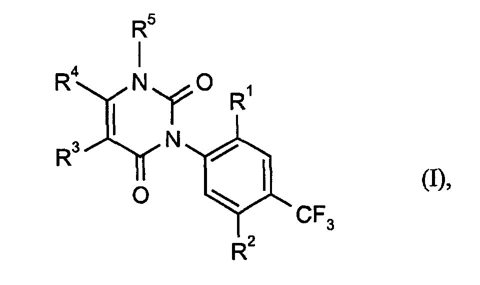

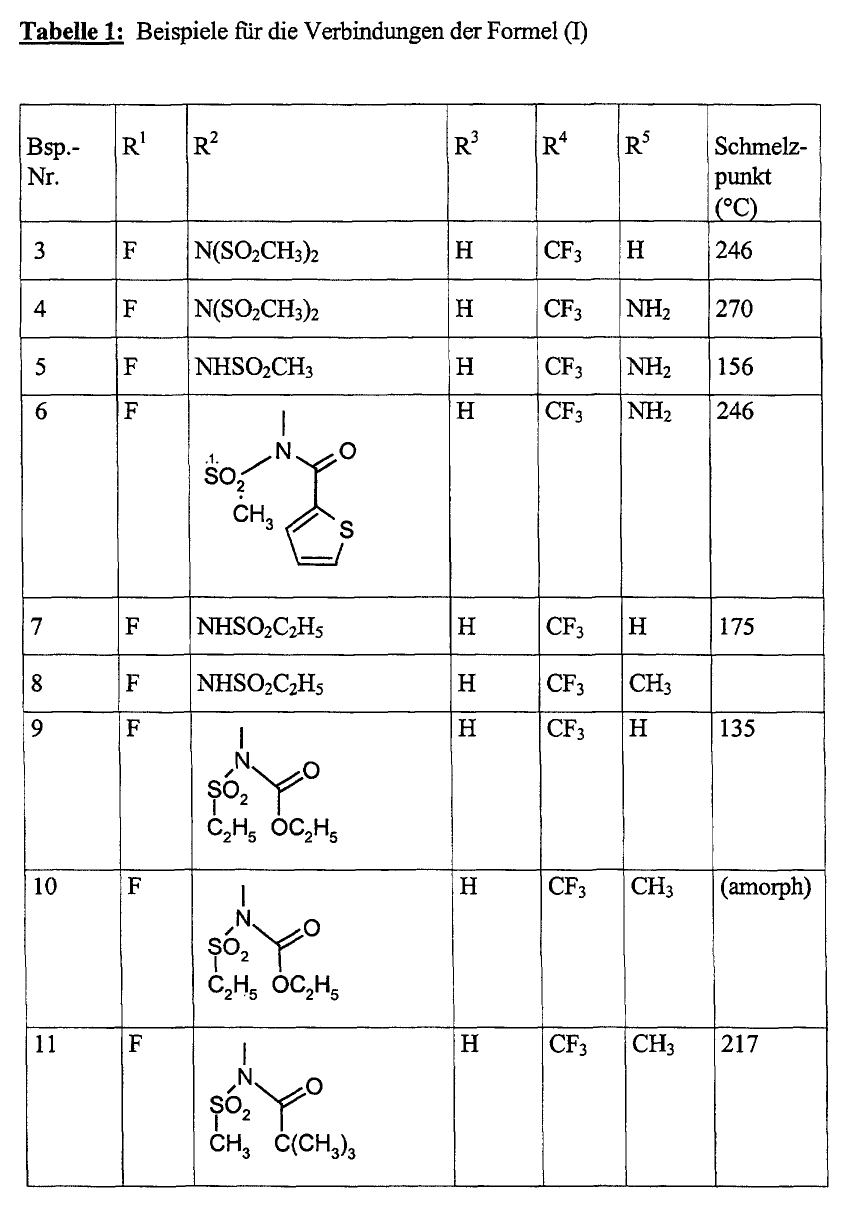

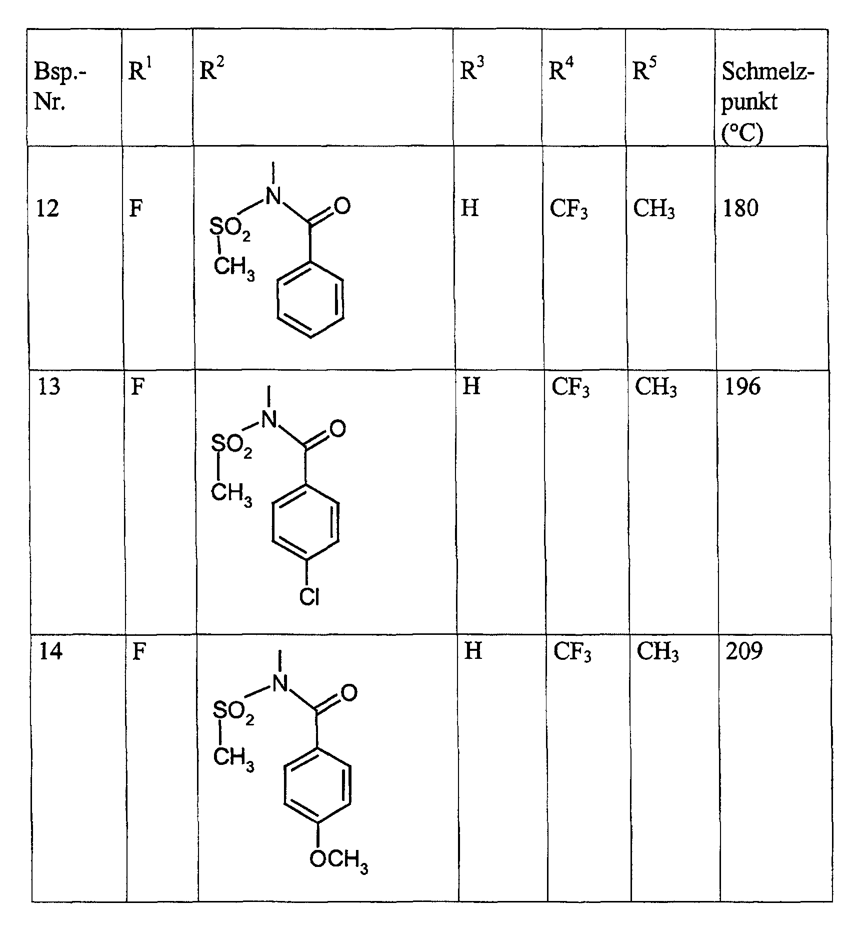

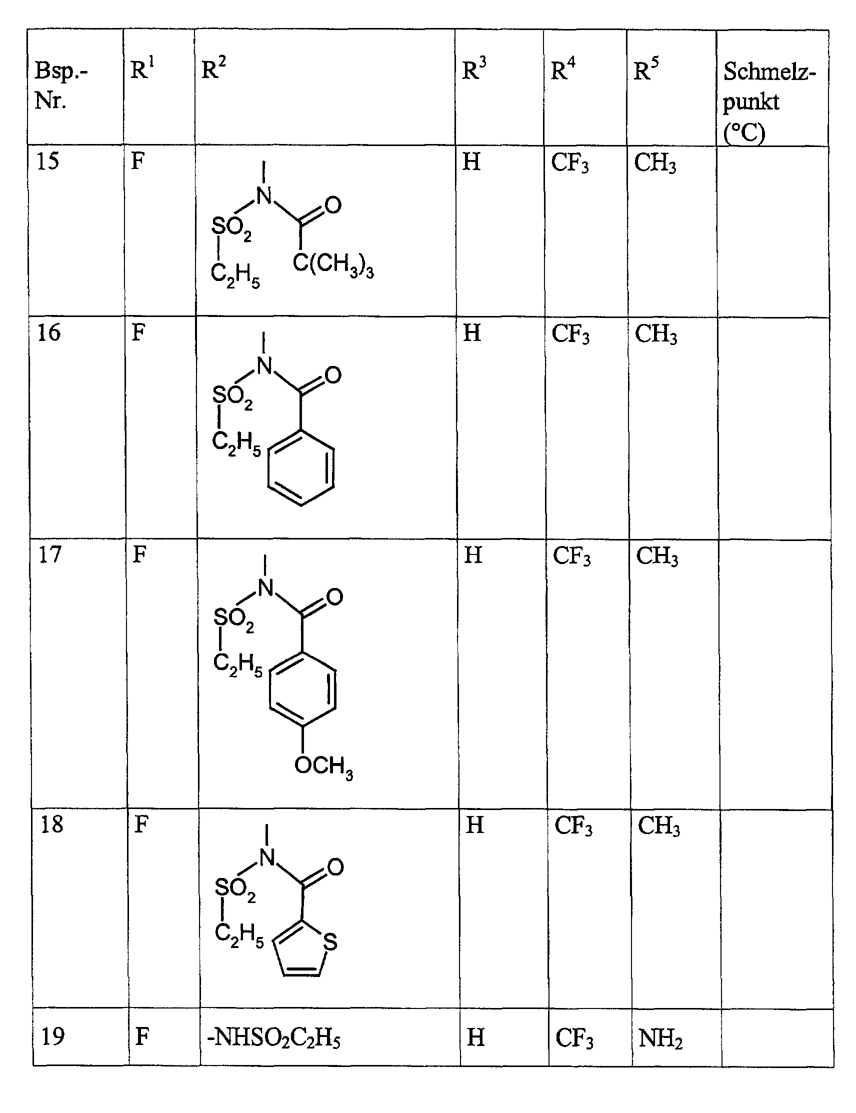

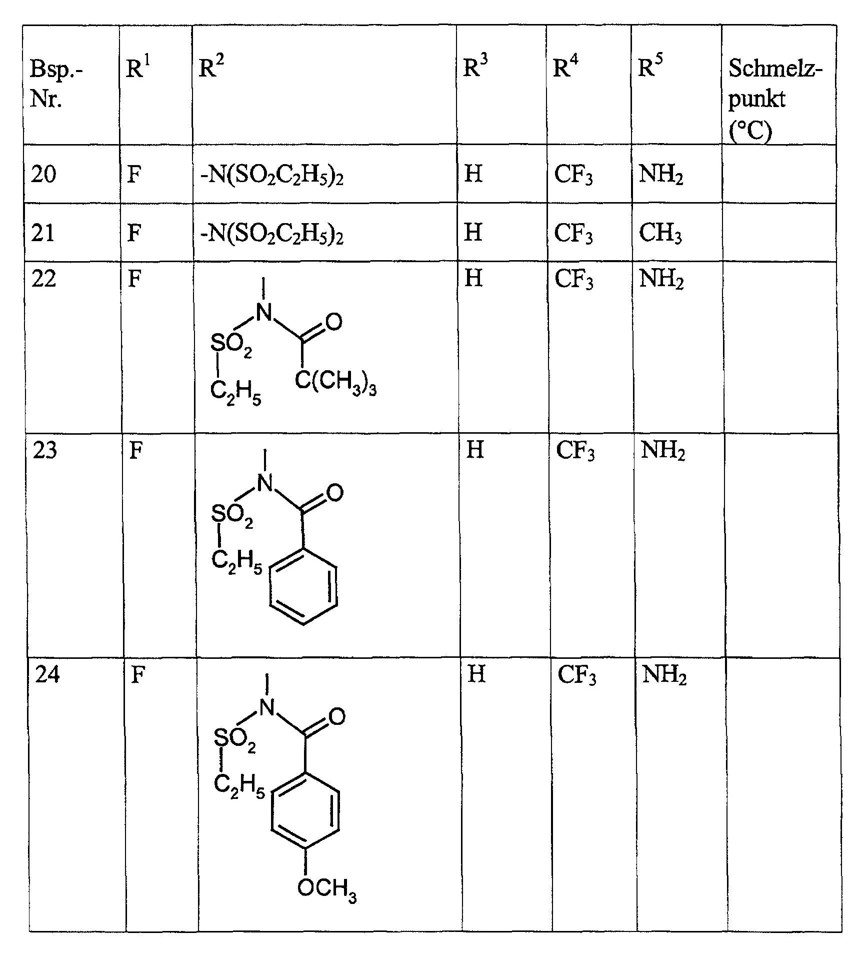

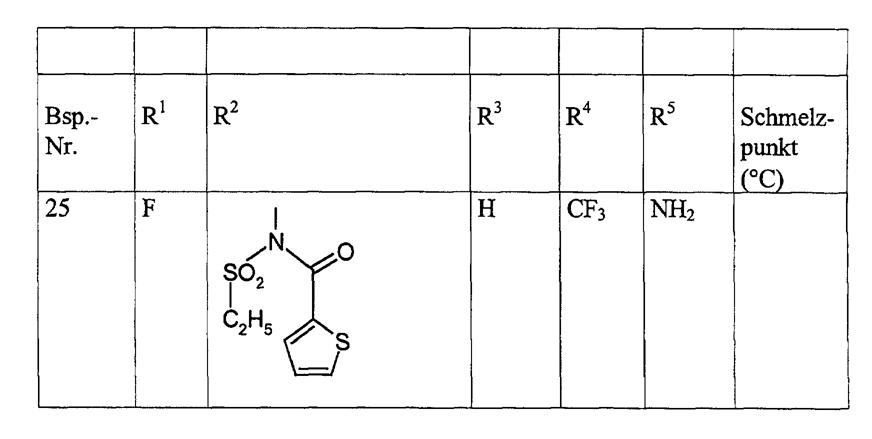

### Anwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung gespritzt, so daß die jeweils gewünschten Wirkstoffmengen pro Flächeneinheit ausgebracht werden. Die Konzentration der Spritzbrühe wird so gewählt, daß in 1000l Wasser/kg die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.
Es bedeuten:
- 0 % =: keine Wirkung (wie unbehandelte Kontrolle)
- 100 % =: totale Vernichtung

In diesem Test zeigen beispielsweise die Verbindungen gemäß Herstellungsbeispiel 1 und 2 bei teilweise guter Verträglichkeit gegenüber Kulturpflanzen, wie z.B. Mais (0 - 10 %), Weizen (0 %), Soja (0 %) und Zuckerrüben (0 %), starke Wirkung gegen Unkräuter, wie Cyperus (80 %), Sinapis (100 %), Abutilon (80 - 100 %), Chenopodium (80 - 100 %), Matricaria (90 - 100 %) und Solanum (90 - 100 %).

### Beispiel B

### Post-emergence-Test

- Löungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000l Wasser/ha die jeweils gewünschten Wirkstoffinengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
- 0 % =: keine Wirkung (wie unbehandelte Kontrolle)
- 100 % =: totale Vernichtung

In diesem Test zeigen beispielsweise die Verbindungen gemäß Herstellungsbeispiel 1 und 2 bei guter Verträglichkeit gegenüber Kulturpflanzen, wie z.B. Mais (0 %), Weizen (0 - 5 %) und Gerste (0 - 10 %), starke Wirkung gegen Unkräuter, wie Sinapis (100 %), Datura (100 %), Ipomoea (100 %), Polygonum (95 %), Solanum (100 %), Abutilon (100 %) und Matricaria (100 %).

## Patentansprüche

1. Substituierte p-Trifluormethylphenyluracile der allgemeinen Formel (I) 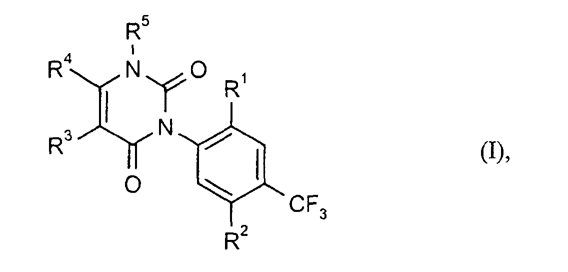 **dadurch gekennzeichnet, daß**
R¹ für Wasserstoff, Cyano, Fluor, Chlor oder Brom steht,
R² für die Gruppierung A¹-A²-A³ steht, worin
A¹ für die Gruppierung -N-A⁴- steht, worin A⁴ für Wasserstoff, Hydroxy, C₁-C₄-Alkyl, C₁-C₄-Alkoxy, Phenyl, C₁-C₄-Alkylsulfonyl oder Phenylsulfonyl steht,
A² für eine Einfachbindung, für O, S, -SO-, -SO₂-, -CO- oder die Gruppierung -N-A⁴- steht, worin A⁴ für Wasserstoff, Hydroxy, C₁-C₄-Alkyl, C₁-C₄-Alkoxy, Phenyl, C₁-C₄-Alkylsulfonyl oder Phenylsulfonyl steht,
A² weiterhin für jeweils gegebenenfalls durch Fluor oder Chlor substituiertes C₁-C₆-Alkandiyl, C₂-C₆-Alkendiyl, C₂-C₆₋Alkindiyl, C₃-C₆-Cycloalkandiyl, C₃-C₆-Cycloalkendiyl oder Phenylen steht,
A³ für Wasserstoff, Hydroxy, Mercapto, Amino, Cyano, Isocyanato, Thiocyanato, Nitro, Carboxy, Carbamoyl, Thiocarbamoyl, Sulfo, Chlorsulfonyl, Halogen, für jeweils gegebenenfalls durch Fluor, Chlor, Brom oder C₁-C₄-Alkoxy substituiertes Alkyl, Alkoxy, Alkylthio, Alkylsulfinyl, Alkylsulfonyl, Alkylamino, Dialkylamino, Alkoxycarbonyl oder Dialkoxy(thio)phosphoryl mit jeweils 1 bis 6 Kohlenstoffatomen in den Alkylgruppen, für jeweils gegebenenfalls durch Fluor, Chlor oder Brom substituiertes Alkenyl, Alkenyloxy, Alkenylamino, Alkylidenamino, Alkenyloxycarbonyl, Alkinyl, Alkinyloxy, Alkinylamino oder Alkinyloxycarbonyl mit jeweils 2 bis 6 Kohlenstoffatomen in den Alkenyl-, Alkyliden- oder Alkinylgruppen, für jeweils gegebenenfalls durch Fluor, Chlor, Brom, Cyano, Carboxy, C₁-C₄-Alkyl und/oder C₁-C₄-Alkoxy-carbonyl substituiertes Cycloalkyl, Cycloalkyloxy, Cycloalkylalkyl, Cycloalkylalkoxy, Cycloalkylidenamino, Cycloalkyloxycarbonyl oder Cycloalkylalkoxycarbonyl mit jeweils 3 bis 6 Kohlenstoffatomen in den Cycloalkylgruppen und gegebenenfalls 1 bis 4 Kohlenstoffatomen in den Alkylgruppen, oder für jeweils gegebenenfalls durch Nitro, Cyano, Carboxy, Fluor, Chlor, Brom, C₁-C₄-Alkyl, C₁-C₄-Halogenalkyl, C₁-C₄-Alkyloxy, C₁-C₄-Halogenalkyloxy und/oder C₁-C₄-Alkoxy-carbonyl substituiertes Phenyl, Phenyloxy, Phenyl- C₁-C₄-alkyl, Phenyl-C₁-C₄-alkoxy, Phenyloxycarbonyl oder Phenyl-C₁-C₄-alkoxycarbonyl, (jeweils gegebenenfalls ganz oder teilweise hydriertes) Pyrrolyl, Pyrazolyl, Imidazolyl, Triazolyl, Furyl, Thienyl, Oxazolyl, Isoxazolyl, Thiazolyl, Isothiazolyl, Oxadiazolyl, Thiadiazolyl, Pyridinyl, Pyrimidinyl, Triazinyl, Pyrazolyl-C₁-C₄-alkyl, Furyl-C₁-C₄-alkyl, Thienyl- C₁-C₄-alkyl, Oxazolyl-C₁-C₄-alkyl, Isoxazol-C₁-C₄-alkyl, Thiazol-C₁-C₄-alkyl, Pyridinyl-C₁-C₄-alkyl, Pyrimidinyl-C₁-C₄-alkyl, Pyrazolylmethoxy, Furylmethoxy, für Perhydropyranyl-methoxy oder Pyridylmethoxy steht,
R³ für Wasserstoff, Fluor, Chlor, Brom oder gegebenenfalls durch Fluor und/oder Chlor substituiertes Alkyl mit 1 bis 4 Kohlenstoffatomen steht,
R⁴ für gegebenenfalls durch Fluor und/oder Chlor substituiertes Alkyl mit 1 bis 4 Kohlenstoffatomen steht, und
R⁵ für Wasserstoff, Amino oder gegebenenfalls durch Hydroxy, Cyano, Fluor, Chlor oder C₁-C₄-Alkoxy substituiertes Alkyl mit 1 bis 4 Kohlenstoffatomen steht.

2. Substituierte p-Trifluormethylphenyluracile der allgemeinen Formel (I) gemäß Anspruch 1, **dadurch gekennzeichnet, daß**
R¹ für Wasserstoff, Fluor oder Chlor steht,
R² für die Gruppierung A¹-A²-A³ steht, worin
A¹ für die Gruppierung -N-A⁴- steht, worin A⁴ für Wasserstoff, Hydroxy, Methyl, Ethyl, n- oder i-Propyl, Methoxy, Ethoxy, n- oder i-Propoxy, Methylsulfonyl oder Ethylsulfonyl steht,
A² für eine Einfachbindung, für O, S, -SO-, -SO₂-, -CO- oder die Gruppierung -N-A⁴- steht, worin A⁴ für Wasserstoff, Hydroxy, Methyl, Ethyl, n- oder i-Propoxy, Methylsulfonyl, Ethylsulfonyl, n- oder i-Propylsulfonyl oder Phenylsulfonyl steht,
A² weiterhin für Methyl, Ethan-1,1-diyl, Ethan-1,2-diyl, Propan-1,1-diyl, Propan-1,2-diyl, Propan-1,3-diyl, Ethen-1,2-diyl, Propen-1,2-diyl, Propen-1,3-diyl, Ethin- 1,2-diyl oder Propin-1,3-diyl steht,
A³ für Wasserstoff, Hydroxy, Amino, Cyano, Nitro, Carboxy, Carbamoyl, Sulfo, Fluor, Chlor, Brom, jeweils gegebenenfalls durch Fluor, Chlor, Methoxy oder Ethoxy substituiertes Methyl, Ethyl, n- oder i-Propyl, n-, i-, s- oder t-Butyl, n-, i-, s- oder t- Pentyl, Methoxy, Ethoxy, n- oder i-Propoxy, n-, i-, s- oder t- Pentyl, Methoxy, Ethoxy, n- oder i-Propoxy, n-, i-, s- oder t-Butoxy, n-, i-, s- oder t-Pentyloxy, Methylthio, Ethylthio, n- oder i- Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i- Propylamino, n-, i-, s- oder t-Butylamino, Dimethylamino, Diethylamino, Methoxycarbonyl, Ethoxycarbonyl, n- oder i- Propoxycarbonyl, Dimethoxyphosphoryl, Diethoxyphosphoryl, Dipropoxyphosphoryl oder Diisopropoxyphosphoryl, für jeweils gegebenenfalls durch Fluor oder Chlor substituiertes Propenyl, Butenyl, Propenyloxy, Butenyloxy, Propenylamino, Butenylamino, Propylidenamino, Butylidenamino, Propenyloxycarbonyl, Butenyloxycarbonyl, Propinyl, Butinyl, Propinyloxy, Butinyloxy, Propinylamino, Butinylamino, Propinyloxycarbonyl oder Butinyloxycarbonyl, für jeweils gegebenenfalls durch Fluor, Chlor, Cyano, Carboxy, Methyl, Ethyl, n- oder i-Propyl, Methoxycarbonyl oder Ethoxycarbonyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopentylidenamino, Cyclohexylidenamino, Cyclopentyloxycarbonyl, Cyclohexyloxycarbonyl, Cyclopentylmethoxycarbonyl oder Cyclohexylmethoxycarbonyl, oder für jeweils gegebenenfalls durch Nitro, Cyano, Carboxy, Fluor, Chlor, Brom, Methyl, Ethyl, n- oder i-Propyl, Trifluormethyl, Methoxy, Ethoxy, n-oder i-Propoxy, Difluormethoxy, Trifluormethoxy, Methoxycarbonyl und/oder Enthoxycarbonyl substituiertes Phenyl, Phenyloxy, Benzyl, Phenylethyl, Benzyloxy, Phenyloxycarbonyl, Benzyloxycarbonyl, (jeweils gegebenenfalls ganz oder teilweise hydriertes) Pyrrolyl, Pyrazolyl, Imidazolyl, Triazolyl, Furyl, Thienyl, Oxazolyl, Isoxazolyl, Thiazolyl, Isothiazolyl, Oxadiazolyl, Thioadiazolyl, Pyridinyl, Pyrimidinyl, Triazinyl, Pyrazolylmethyl, Furylmethyl, Thienylmethyl, Oxazolylmethyl, Isoxazolmethyl, Thiazolmethyl, Pyridinylmethyl, Pyrimidinylmethyl, Pyrazolylmethoxy, Furylmethoxy oder Pyridylmethoxy steht,
R³ für Wasserstoff, Fluor, Chlor oder jeweils gegebenenfalls durch Fluor und/oder Chlor substituiertes Methyl oder Ethyl steht,
R⁴ für jeweils durch Fluor und/oder Chlor substituiertes Methyl oder Ethyl steht, und
R⁵ für Wasserstoff, Amino oder jeweils gegebenenfalls durch Hydroxy, Cyano, Fluor, Chlor, Methoxy oder Ethoxy substituiertes Methyl oder Ethyl steht.

3. Verfahren zur Herstellung substituierter p-Trifluormethylphenyluracile der allgemeinen Formel (I) 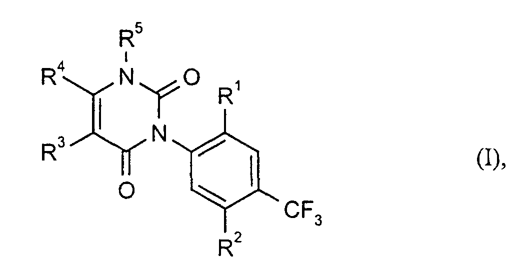 in welcher
R¹, R², R³, R⁴ und R⁵ die in Anspruch 1 genannten Bedeutungen haben, **dadurch gekennzeichnet, daß** man
Aminoalkensäureester der allgemeinen Formel (II) 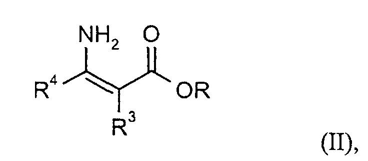 in welcher
R³ und R⁴ die oben angegebenen Bedeutungen haben und
R für Alkyl, Aryl oder Arylalkyl steht,
mit p-Trifluormethylphenylisocyanaten der allgemeinen Formel (III) 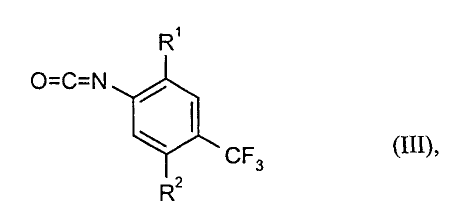 in welcher
R¹ und R² die oben angegebenen Bedeutungen haben,
oder mit p-Trifluormethylphenylurethanen (p-Trifluormethylphenylcarbamaten) der allgemeinen Formel (IV) 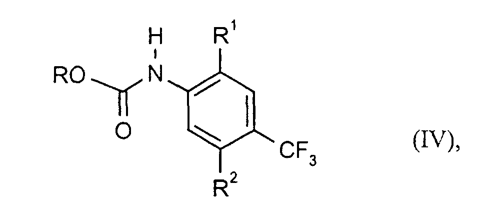 in welcher
R, R¹ und R² die oben angegebenen Bedeutungen haben, umsetzt.

4. Herbizide Mittel, **gekennzeichnet durch** einen Gehalt an mindestens einem p-Trifluormethylphenyluracil der Formel (I) gemäß dem Anspruch 1.

5. Verfahren zur Bekämpfung von unerwünschten Pflanzen, **dadurch gekennzeichnet, daß** man p-Trifluormethylphenyluracile der Formel (I) gemäß dem Anspruch 1 auf unerwünschte Pflanzen und/oder ihren Lebensraum einwirken läßt.

6. Verwendung von p-Trifluormethylphenyluracilen der Formel (I) gemäß dem Anspruch 1 zur Bekämpfung von unerwünschten Pflanzen.

7. Verfahren zur Herstellung von herbiziden Mitteln, **dadurch gekennzeichnet, daß** man p-Trifluormethylphenyluracile der Formel (I) gemäß dem Anspruch 1 mit Streckmitteln und/oder oberflächenaktiven Substanzen vermischt.

8. p-Trifluormethylphenylcarbamate der allgemeinen Formel (IV) 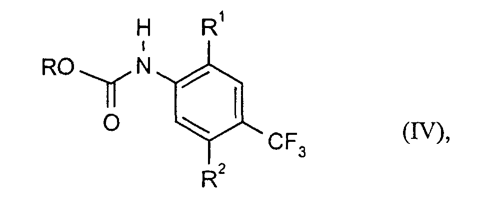 in welcher
R¹ und R² die in Anspruch 1 genannten Bedeutungen haben und R für Alkyl, Aryl oder Arylalkyl steht.

## Claims

1. Substituted p-trifluoromethylphenyluracils of the general formula (I) 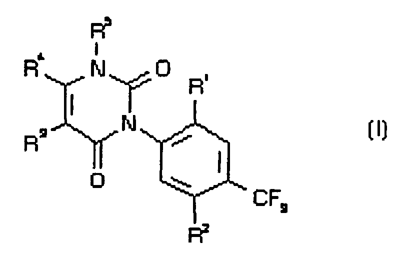 **characterized in that**
R¹ represents hydrogen, cyano, fluorine, chlorine or bromine,
R² represents the grouping A¹-A²-A³ in which
A¹ represents a single bond, represents O, S, -SO-, -SO₂-, -CO- or the grouping -N-A⁴- in which A⁴ represents hydrogen, hydroxyl, C₁-C₄-alkyl, C₁-C₄-alkoxy, phenyl, C₁-C₄-alkylsulphonyl or phenylsulphonyl,
A² represents a single bond, represents O, S, -SO-, -SO₂-, -CO- or the grouping -N-A⁴- in which A⁴ represents hydrogen, hydroxyl, C₁-C₄-alkyl, C₁-C₄-alkoxy, phenyl, C₁-C₄-alkylsulphonyl or phenylsulphonyl,
A² furthermore represents C₁-C₆-alkanediyl, C₂-C₆-alkenediyl, C₂-C₆-alkinediyl, C₃-C₆-cycloalkanediyl, C₃-C₆-cycloalkenediyl or phenylene, each of which is optionally substituted by fluorine or chlorine,
A³ represents hydrogen, hydroxyl, mercapto, amino, cyano, isocyanato, thiocyanato, nitro, carboxyl, carbamoyl, thiocarbamoyl, sulpho, chlorosulphonyl, halogen, represents alkyl, alkoxy, alkylthio, alkylsulphinyl, alkylsulphonyl, alkylamino, dialkylamino, alkoxycarbonyl or dialkoxy(thio)phosphoryl having in each case 1 to 6 carbon atoms in the alkyl groups and being in each case optionally substituted by fluorine, chlorine, bromine or C₁-C₄-alkoxy, represents alkenyl, alkenyloxy, alkenylamino, alkylideneamino, alkenyloxycarbonyl, alkinyl, alkinyloxy, alkinylamino or alkinyloxycarbonyl having in each case 2 to 6 carbon atoms in the alkenyl, alkylidene or alkinyl groups and being in each case optionally substituted by fluorine, chlorine or bromine, represents cycloalkyl, cycloalkyloxy, cycloalkylalkyl, cycloalkylalkoxy, cycloalkylideneamino, cycloalkyloxycarbonyl or cycloalkylalkoxycarbonyl having in each case 3 to 6 carbon atoms in the cycloalkyl groups and optionally 1 to 4 carbon atoms in the alkyl groups and being in each case optionally substituted by fluorine, chlorine, bromine, cyano, carboxyl, C₁-C₄-alkyl and/or C₁-C₄-alkoxycarbonyl, or represents phenyl, phenyloxy, phenyl-C₁-C₄-alkyl, phenyl-C₁-C₄-alkoxy, phenyloxycarbonyl or phenyl-C₁-C₄-alkoxy-carbonyl, each of which is optionally substituted by nitro, cyano, carboxyl, fluorine, chlorine, bromine, C₁-C₄-alkyl, C₁-C₄-halogenoalkyl, C₁-C₄-alkyloxy, C₁-C₄-halogenoalkyloxy and/or C₁-C₄-alkoxy-carbonyl, represents (in each case optionally fully or partially hydrogenated) pyrrolyl, pyrazolyl, imidazolyl, triazolyl, furyl, thienyl, oxazolyl, isoxazolyl, thiazolyl, isothiazolyl, oxadiazolyl, thiadiazolyl, pyridinyl, pyrimidinyl, triazinyl, pyrazolyl-C₁-C₄-alkyl, furyl-C₁-C₄-alkyl, thienyl-C₁-C₄-alkyl, oxazolyl-C₁-C₄-alkyl, isoxazol-C₁-C₄-alkyl, thiazole-C₁-C₄-alkyl, pyridinyl-C₁-C₄-alkyl, pyrimidinyl-C₁-C₄-alkyl, pyrazolylmethoxy, furyl-methoxy, represents perhydropyranyl-methoxy or pyridylmethoxy,
R³ represents hydrogen, fluorine, chlorine, bromine or optionally fluorine- and/or chlorine-substituted alkyl having 1 to 4 carbon atoms,
R⁴ represents optionally fluorine- and/or chlorine-substituted alkyl having 1 to 4 carbon atoms, and
R⁵ represents hydrogen, amino or optionally hydroxyl-, cyano-, fluorine-, chlorine- or C₁-C₄-alkoxy-substituted alkyl having 1 to 4 carbon atoms.

2. Substituted p-trifluoromethylphenyluracils of the general formula (I) according to Claim 1, **characterized in that**
R¹ represents hydrogen, fluorine or chlorine,
R² represents the grouping A¹-A²-A³ in which
A¹ represents the grouping -N-A⁴- in which A⁴ represents hydrogen, hydroxyl, methyl, ethyl, n- or i-propyl, methoxy, ethoxy, n- or i-propoxy, methylsulphonyl or ethylsulphonyl,
A² represents a single bond, represents O, S, -SO-, -SO₂-, -CO- or the grouping -N-A⁴- in which A⁴ represents hydrogen, hydroxyl, methyl, ethyl, n- or i-propyl, methylsulphonyl, ethylsulphonyl, n- or i- propylsulphonyl or phenylsulphonyl,
A² furthermore represents methyl, ethane-1,1-diyl, ethane-1,2-diyl, propane-1,1-diyl, propane-1,2-diyl, propane-1,3-diyl, ethene-1,2-diyl, propene-1,2-diyl, propene-1,3-diyl, ethine-1,2-diyl or propine-1,3-diyl,
A³ represents hydrogen, hydroxyl, amino, cyano, nitro, carboxyl, carbamoyl, sulpho, fluorine, chlorine, bromine, represents methyl, ethyl, n- or i-propyl, n-, i-, s- or t-butyl, n-, i-, s- or t- pentyl, methoxy, ethoxy, n- or i-propoxy, n-, i-, s- or t-pentyl, methoxy, ethoxy, n- or i-propoxy, n-, i-, s- or t-butoxy, n-, i-, s- or t-pentyloxy, methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, methylamino, ethylamino, n- or i-propylamino, n-, i-, s- or t-butylamino, dimethylamino, diethylamino, methoxycarbonyl, ethoxycarbonyl, n- or i-propoxycarbonyl, dimethoxyphosphoryl, diethoxyphosphoryl, dipropoxyphosphoryl or diisopropoxyphosphoryl, each of which is optionally substituted by fluorine, chlorine, methoxy or ethoxy, represents propenyl, butenyl, propenyloxy, butenyloxy, propenylamino, butenylamino, propylideneamino, butylideneamino, propenyloxycarbonyl, butenyloxycarbonyl, propinyl, butinyl, propinyloxy, butinyloxy, propinylamino, butinylamino, propinyloxycarbonyl or butinyloxycarbonyl, each of which is optionally substituted by fluorine or chlorine, represents cyclopropyl, cyclobutyl, cyclopentyl, cyclohexyl, cyclopropyloxy, cyclobutyloxy, cyclopentyloxy, cyclohexyloxy, cyclopropylmethyl, cyclobutylmethyl, cyclopentylmethyl, cyclohexylmethyl, cyclopropylmethoxy, cyclobutylmethoxy, cyclopentylmethoxy, cyclohexylmethoxy, cyclopentylideneamino, cyclohexylideneamino, cyclopentyloxycarbonyl, cyclohexyloxycarbonyl, cyclopentylmethoxycarbonyl or cyclohexylmethoxycarbonyl, each of which is optionally substituted by fluorine, chlorine, cyano, carboxyl, methyl, ethyl, n- or i-propyl, methoxycarbonyl or ethoxycarbonyl, or represents phenyl, phenyloxy, benzyl, phenylethyl, benzyloxy, phenyloxycarbonyl, benzyloxycarbonyl, each of which is optionally substituted by nitro, cyano, carboxyl, fluorine, chlorine, bromine, methyl, ethyl, n- or i-propyl, trifluoromethyl, methoxy, ethoxy, n- or i-propoxy, difluoromethoxy, trifluoromethoxy, methoxycarbonyl and/or ethoxycarbonyl, represents (in each case optionally fully or partially hydrogenated) pyrrolyl, pyrazolyl, imidazolyl, triazolyl, furyl, thienyl, oxazolyl, isoxazolyl, thiazolyl, isothiazolyl, oxadiazolyl, thioadiazolyl, pyridinyl, pyrimidinyl, triazinyl, pyrazolylmethyl, furylmethyl, thienylmethyl, oxazolylmethyl, isoxazolmethyl, thiazolemethyl, pyridinylmethyl, pyrimidinylmethyl, pyrazolylmethoxy, furylmethoxy or pyridylmethoxy,
R³ represents hydrogen, fluorine, chlorine or in each case optionally fluorine- and/or chlorinesubstituted methyl or ethyl,
R⁴ represents in each case fluorine- and/or chlorine-substituted methyl or ethyl, and
R⁵ represents hydrogen, amino or in each case optionally hydroxyl-, cyano-, fluorine-, chlorine-, methoxy- or ethoxy-substituted methyl or ethyl.

3. Process for preparing substituted p-trifluoromethylphenyluracils of the general formula (I) 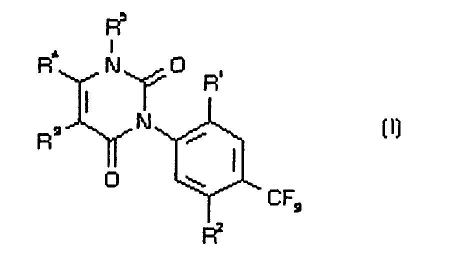 in which
R¹, R², R³, R⁴ and R⁵ are each as defined in Claim 1, **characterized in that**
aminoalkenoic esters of the general formula (II) 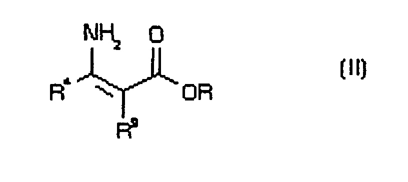 in which
R³ and R⁴ are each as defined above and
R represents alkyl, aryl or arylalkyl,
are reacted with p-trifluoromethylphenyl isocyanates of the general formula (III) 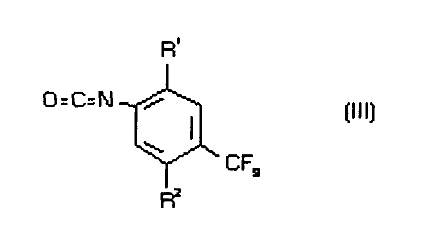 in which
R¹ and R² are each as defined above
or with p-trifluoromethylphenylurethanes (p-trifluoromethylphenylcarbamates) of the general formula (IV) 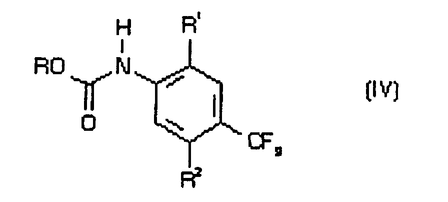 in which
R, R¹ and R² are each as defined above.

4. Herbicidal compositions, **characterized in that** they contain at least one p-trifluoromethylphenyluracil of the formula (I) according to Claim 1.

5. Method for controlling undesirable plants, **characterized in that** p-trifluoromethylphenyluracils of the formula (I) according to Claim 1 are allowed to act on undesirable plants and/or their habitat.

6. Use of p-trifluoromethylphenyluracils of the formula (I) according to Claim 1 for controlling undesirable plants.

7. Process for preparing herbicidal compositions, **characterized in that** p-trifluoromethylphenyluracils of the formula (I) according to Claim 1 are mixed with extenders and/or surfactants.

8. p-Trifluoromethylphenylcarbamates of the general formula (IV) 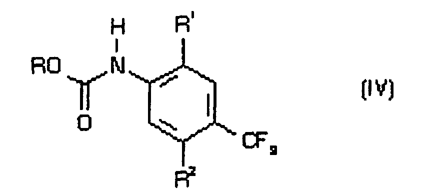 in which
R¹ and R² are each as defined in Claim 1 and R represents alkyl, aryl or arylalkyl.

## Revendications

1. p-Trifluorométhylphényluraciles substitués répondant à la forme générale (I) 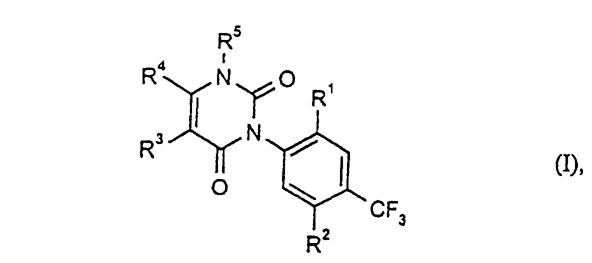 **caractérisés en ce que**
R¹ représente un atome d'hydrogène, un groupe cyano, un atome de fluor, un atome de chlore ou un atome de brome,
R² représente le groupement A¹-A²-A³ dans lequel
A¹ représente le groupement -N-A⁴- dans lequel A⁴ représente un atome d'hydrogène, un groupe hydroxyle, un groupe alkyle en C₁-C₄, un groupe alcoxy en C₁-C₄, un groupe phényle, un groupe alkyl(en C₁-C₄)sulfonyle ou un groupe phénylsulfonyle,
A² représente une liaison simple ; représente un atome d'oxygène, un atome de soufre, un groupe -SO-, un groupe -SO₂-, un groupe -CO- ou le groupement -N-A⁴- dans lequel A⁴ représente un atome d'hydrogène, un groupe hydroxyle, un groupe alkyle en C₁-C₄, un groupe alcoxy en C₁-C₄, un groupe phényle, un groupe alkyl(en C₁-C₄)sulfonyle ou un groupe phényl-sulfonyle,
A² représente en outre un groupe alcane(en C₁-C₆)diyle, un groupe alcène(en C₂-C₆)diyle, un groupe alcyne(en C₂-C₆)diyle, un groupe cycloalcane(en C₃-C₆)diyle, un groupe cycloalcène(en C₃-C₆)diyle, ou un groupe phénylène, chacun de ces groupes portant, le cas échéant, un ou plusieurs substituants identiques ou différents fluoro ou chloro,
A³ représente un atome d'hydrogène, un groupe hydroxyle, un groupe mercapto, un groupe amino, un groupe cyano, un groupe isocyanato, un groupe thiocyanato, un groupe nitro, un groupe carboxyle, un groupe carbamoyle, un groupe thiocarbamoyle, un groupe sulfo, un groupe chlorosulfonyle, un atome d'halogène ; représente un groupe alkyle, un groupe alcoxy, un groupe alkylthio, un groupe alkylsulfinyle, un groupe alkylsulfonyle, un groupe alkylamino, un groupe dialkylamino, un groupe alcoxycarbonyle ou un groupe dialcoxy(thio)phosphoryle, chacun de ces groupes contenant de 1 à 6 atomes de carbone dans les groupes alkyle et chacun de ces groupes portant, le cas échéant, un ou plusieurs substituants identiques ou différents fluoro, chloro, bromo ou alcoxy en C₁-C₄ ; représente un groupe alcényle, un groupe alcényloxy, un groupe alcénylamino, un groupe alkylidène-amino, un groupe alcényloxycarbonyle, un groupe alcynyle, un groupe alcynyloxy, un groupe alcynylamino ou un groupe alcynyloxycarbonyle, chacun de ces groupes contenant de 2 à 6 atomes de carbone dans les groupes alcényle, alkylidène ou alcynyle et chacun de ces groupes portant, le cas échéant, un ou plusieurs substituants identiques ou différents fluoro, chloro ou bromo ; représente un groupe cycloalkyle, un groupe cycloalkyloxy, un groupe cycloalkylalkyle, un groupe cycloalkylalcoxy, un groupe cycloalkylidène-amino, un groupe cycloalkyloxycarbonyle ou un groupe cycloalkylalcoxycarbonyle, chacun de ces groupes contenant de 3 à 6 atomes de carbone dans les groupes cycloalkyle et le cas échéant de 1 à 4 atomes de carbone dans les groupes alkyle et chacun de ces groupes portant, le cas échéant, un ou plusieurs substituants identiques ou différents fluoro, chloro, bromo, cyano, carboxyle, alkyle en C₁-C₄ et/ou alcoxy(en C₁-C₄)carbonyle ; ou représente un groupe phényle, un groupe phényloxy, un groupe phénylalkyle en C₁-C₄, un groupe phénylalcoxy en C₁-C₄, un groupe phényloxycarbonyle où un groupe phénylalcoxy(en C₁-C₄)carbonyle, chacun de ces groupes portant, le cas échéant, un ou plusieurs substituants identiques ou différents nitro, cyano, carboxyle, fluoro, chloro, bromo, alkyle en C₁-C₄, halogénalkyle en C₁-C₄, alkyl(en C₁-C₄)oxy, halogénalkyl(en C₁-C₄)oxy et/ou alcoxy(en C₁-C₄)carbonyle ; un groupe pyrrolyle, un groupe pyrazolyle, un groupe imidazolyle, un groupe triazolyle, un groupe furyle, un groupe thiényle, un groupe oxazolyle, un groupe isoxazolyle, un groupe thiazolyle, un groupe isothiazolyle, un groupe oxadiazolyle, un groupe thiadiazolyle, un groupe pyridinyle, un groupe pyrimidinyle, un groupe triazinyle, un groupe pyrazolylalkyle en C₁-C₄, un groupe furylalkyle en C₁-C₄, un groupe thiénylalkyle en C₁-C₄, un groupe oxazolylalkyle en C₁-C₄, un groupe isoxazolalkyle en C₁-C₄, un groupe thiazolalkyle en C₁-C₄, un groupe pyridinylalkyle en C₁-C₄, un groupe pyrimidinylalkyle en C₁-C₄, un groupe pyrazolylméthoxy, un groupe furylméthoxy (chacun de ces groupes étant le cas échéant complètement ou partiellement hydrogénés), chacun de ces groupes portant également, le cas échéant, un ou plusieurs substituants identiques ou différents nitro, cyano, carboxyle, fluoro, chloro, bromo, alkyle en C₁-C₄, halogénalkyle en C₁-C₄, alkyl(en C₁-C₄)oxy, halogénalkyl(en C₁-C₄)oxy et/ou alcoxy(en C₁-C₄)carbonyle ; représente un groupe perhydropyranylméthoxy ou un groupe pyridylméthoxy,
R³ représente un atome d'hydrogène, un atome de fluor, un atome de chlore, un atome de brome ou représente un groupe alkyle contenant de 1 à 4 atomes de carbone portant le cas échéant un ou plusieurs substituants identiques ou différents fluoro et/ou chloro,
R⁴ représente un groupe alkyle contenant de 1 à 4 atomes de carbone portant le cas échéant un ou plusieurs substituants identiques ou différents fluoro et/ou chloro, et
R⁵ représente un atome d'hydrogène, un groupe amino ou représente un groupe alkyle contenant de 1 à 4 atomes de carbone portant le cas échéant un ou plusieurs substituants identiques ou différents hydroxyle, cyano, fluoro, chloro ou alcoxy en C₁-C₄.

2. p-Trifluorométhylphényluraciles substitués répondant à la formule générale (I) selon la revendication 1, **caractérisés en ce que**
R¹ représente un atome d'hydrogène, un atome de fluor ou un atome de chlore,
R² représente le groupement A¹-A²-A³ dans lequel
A¹ représente le groupement -N-A⁴- dans lequel A⁴ représente un atome d'hydrogène, un groupe hydroxyle, un groupe méthyle, un groupe éthyle, un groupe n- ou i-propyle un groupe méthoxy, un groupe éthoxy, un groupe n- ou i- propoxy, un groupe méthylsulfonyle ou un groupe éthylsulfonyle,
A² représente une liaison simple ; représente un atome d'oxygène, un atome de soufre, un groupe -SO-, un groupe -SO₂-, un groupe -CO- ou le groupement -N-A⁴- dans lequel A⁴ représente un atome d'hydrogène, un groupe hydroxyle, un groupe méthyle, un groupe éthyle, un groupe n- ou i-propoxy, un groupe méthylsulfonyle, un groupe éthylsulfonyle, un groupe n- ou i-propylsulfonyle ou un groupe phénylsulfonyle,
A² représente en outre un groupe méthyle, un groupe éthane-1,1-diyle, un groupe éthane-1,2-diyle, un groupe propane-1,1-diyle, un groupe propane-1,2-diyle, un groupe propane-1,3-diyle, un groupe éthène-1,2-diyle, un groupe propène-1,3-diyle, un groupe propène-1,2-diyle, un groupe éthyne-1,2-diyle ou un groupe propyne-1,3-diyle,
A³ représente un atome d'hydrogène, un groupe hydroxyle, un groupe amino, un groupe cyano, un groupe nitro, un groupe carboxyle, un groupe carbamoyle, un groupe sulfo, un atome de fluor, un atome de chlore, un atome de brome ; représente un groupe méthyle, un groupe éthyle, un groupe n- ou i-propyle, un groupe n-, i-, s- ou t-butyle, un groupe n-, i-, s- ou t-pentyle, un groupe méthoxy, un groupe éthoxy, un groupe n- ou i-propoxy, un groupe n-, i-, s- ou t-butoxy, un groupe n-, i-, s- ou t-pentyloxy, un groupe méthylthio, un groupe éthylthio, un groupe n- ou i-propylthio, un groupe n-, i-, s- ou t-butylthio, un groupe méthylsulfinyle, un groupe éthylsulfinyle, un groupe n- ou i-propylsulfinyle, un groupe méthylsulfonyle, un groupe éthylsulfonyle, un groupe n- ou i-propylsulfonyle, un groupe méthylamino, un groupe éthylamino, un groupe n- ou i- propylamino, un groupe n-, i-, s- ou t- butylamino, un groupe diméthylamino, un groupe diéthylamino, un groupe méthoxycarbonyle, un groupe éthoxycarbonyle, un groupe n- ou i-propoxycarbonyle, un groupe diméthoxyphosphoryle, un groupe diéthoxyphosphoryle, un groupe dipropoxyphosphoryle ou un groupe diisopropoxy- phosphoryle, chacun de ces groupes portant, le cas échéant, un ou plusieurs substituants identiques différents fluoro, chloro, méthoxy ou éthoxy ; représente un groupe propényle, un groupe butényle, un groupe propényloxy, un groupe butényloxy, un groupe propénylamino, un groupe butényl-amino, un groupe propylidène-amino, un groupe butylidène-amino, un groupe propényloxycarbonyle, un groupe butényloxycarbonyle, un groupe propynyle, un groupe butynyle, un groupe propynyloxy, un groupe butynyloxy, un groupe propynylamino, un groupe butynylamino, un groupe propynyloxycarbonyle ou un groupe butynyloxycarbonyle, chacun de ces groupes portant, le cas échéant, un ou plusieurs substituants identiques ou différents fluoro ou chloro ; représente un groupe cyclopropyle, un groupe cyclobutyle, un groupe cyclopentyle, un groupe cyclohexyle, un groupe cyclopropyloxy, un groupe cyclobutyloxy, un groupe cyclopentyloxy, un groupe cyclohexyloxy, un groupe cyclopropylméthyle, un groupe cyclobutylméthyle, un groupe cyclopentylméthyle, un groupe cyclohexylméthyle, un groupe cyclopropylméthoxy, un groupe cyclobutylméthoxy, un groupe cyclopentylméthoxy, un groupe cyclohexylméthoxy, un groupe cyclopentylidène-amino, un groupe cyclohexylidène-amino, un groupe cyclopentyl-oxycarbonyle, un groupe cyclohexyloxycarbonyle, un groupe cyclopentylméthoxycarbonyle ou un groupe cyclohexylméthoxycarbonyle, chacun de ces groupes portant, le cas échéant, un ou plusieurs substituants fluoro, chloro, cyano, carboxyle, méthyle, éthyle, n- ou i- propyle, méthoxycarbonyle ou éthoxycarbonyle ; ou représente un groupe phényle, un groupe phényloxy, un groupe benzyle, un groupe phényléthyle, un groupe benzyloxy, un groupe phényloxycarbonyle, un groupe benzyloxycarbonyle, chacun de ces groupes portant, le cas échéant un ou plusieurs substituants identiques ou différents nitro, cyano, carboxyle, fluoro, chloro, bromo, méthyle, éthyle, n- ou i-propyle, trifluorométhyle, méthoxy, éthoxy, n- ou i-propoxy, difluorométhoxy, trifluorométhoxy, méthoxycarbonyle et/ou éthoxycarbonyle ; un groupe pyrrolyle, un groupe pyrazolyle, un groupe imidazolyle, un groupe triazolyle, un groupe furyle, un groupe thiényle, un groupe oxazolyle, un groupe isoxazolyle, un groupe thiazolyle, un groupe isothiazolyle, un groupe oxadiazolyle, un groupe thiadiazolyle, un groupe pyridinyle, un groupe pyrimidinyle, un groupe triazinyle, un groupe pyrazolylméthyle, un groupe furylméthyle, un groupe thiénylméthyle, un groupe oxazolylméthyle, un groupe isoxazolméthyle, un groupe thiazolméthyle, un groupe pyridinylméthyle, un groupe pyrimidinylméthyle, un groupe pyrazolylméthoxy, un groupe furylméthoxy ou un groupe pyridylméthoxy (chacun de ces groupés étant, le cas échéant, complètement ou partiellement hydrogénés), chacun de ces groupes portant également, le cas échéant, un ou plusieurs substituants identiques ou différents nitro, cyano, carboxyle, fluoro, chloro, bromo, méthyle, éthyle, n- ou i-propyle, trifluorométhyle, méthoxy, éthoxy, n- ou i-propoxy, difluorométhoxy, trifluorométhoxy, méthoxycarbonyle et/ou éthoxycarbonyle,
R³ représente un atome d'hydrogène, un atome de fluor, un atome de chlore ou représente un groupe méthyle ou un groupe éthyle portant le cas échéant un ou plusieurs substituants identiques ou différents fluoro et/ou chloro,
R⁴ représente un groupe méthyle ou un groupe éthyle portant le cas échéant un ou plusieurs substituants identiques ou différents fluoro et/ou chloro, et
R⁵ représente un atome d'hydrogène, un groupe amino ou représente un groupe méthyle ou un groupe éthyle portant le cas échéant un ou plusieurs substituants identiques ou différents hydroxyle, cyano, fluoro, chloro, méthoxy ou éthoxy.

3. Procédé pour la préparation de p-trifluorométhylphényluraciles répondant à la formule générale (I) 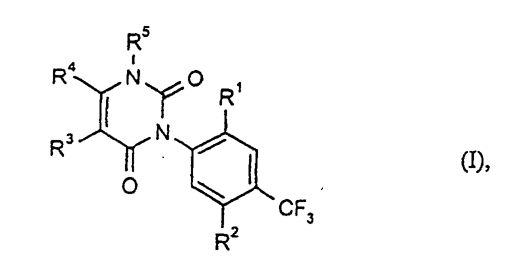 dans laquelle
R¹, R², R³, R⁴ et R⁵ ont les significations indiquées à la revendication 1, **caractérisé en ce qu'**on fait réagir
des esters aminoalcéniques répondant à la formule générale (II) 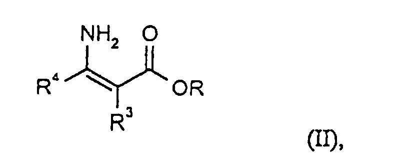 dans laquelle
R³ et R⁴ ont les significations indiquées ci-dessus et
R représente un groupe alkyle, un groupe aryle ou un groupe arylalkyle,
avec des p-trifluorométhylphénylisocyanates répondant à la formule générale (III) 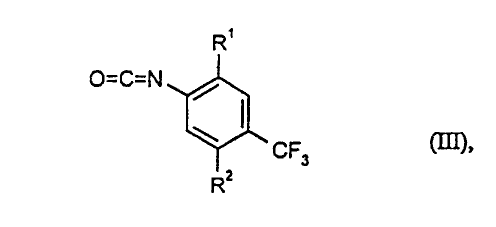 dans laquelle
R¹ et R² ont les significations indiquées ci-dessus,
ou avec des p-trifluorométhylphényluréthanes (p-trifluorométhylphénylcarbamates) répondant à la formule générale (IV) 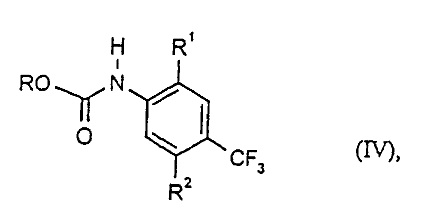 dans laquelle
R, R¹ et R² ont les significations indiquées ci-dessus.

4. Agents herbicides, **caractérisés par** une teneur en au moins un p-trifluorométhylphényluracile répondant à la formule (I) selon la revendication 1.

5. Procédé pour lutter contre des plantes indésirables, **caractérisé en ce qu'**on laisse agir des p-trifluorométhylphényluraciles répondant à la formule (I) selon la revendication 1 sur des plantes non désirées et/ou sur leur biotope.

6. Utilisation de p-trifluorométhylphényluraciles répondant à la formule (I) selon la revendication 1 pour lutter contre des plantes non désirées.

7. Procédé pour la préparation d'agents herbicides, **caractérisé en ce qu'**on mélange des p-trifluorométhylphényluraciles répondant à la formule (I) selon la revendication 1 avec des diluants et/ou avec des substances tensioactives.

8. p-Trifluorométhylphénylcarbamates répondant à la formule générale (IV) 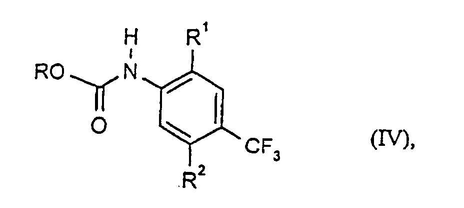 dans laquelle
R¹ et R² ont les significations indiquées à la revendication 1 et R représente un groupe alkyle, un groupe aryle ou un groupe arylalkyle.
